(19) Europäisches Patentamt
European Patent Office
Office européen des brevets

(11) Publication number: **0 220 951 A2**

# (12) EUROPEAN PATENT APPLICATION

(21) Application number: 86308283.0

(22) Date of filing: 24.10.86

(51) Int. Cl.⁴: C 12 P 1/00, C 12 P 19/04, C 12 P 19/02, C 12 N 1/32, C 07 B 59/00 // (C12P1/00, C12R1:00)

(30) Priority: 25.10.85 US 791639

(43) Date of publication of application: 06.05.87 Bulletin 87/19

(84) Designated Contracting States: BE DE FR GB IT NL

(71) Applicant: CELANESE CORPORATION, 1211 Avenue of the Americas, New York New York 10036 (US)

(72) Inventor: Stirling, David I., 4 Lois Place, Fanwood New Jersey (US)
Inventor: Hagedorn, Scott R., 156 Netherwood, North Plainfield New Jersey (US)

(74) Representative: De Minvielle-Devaux, Ian Benedict Peter et al, CARPMAELS & RANSFORD 43, Bloomsbury Square, London WC1A 2RA (GB)

## (54) Biochemicals labelled with stable isotopes.

(57) This invention provides a bioconversion process for producing stable isotope labelled biochemicals.

In one embodiment, Methylophilus viscogenes strain ATCC 39893 is cultivated with $^{13}C$-methanol as the sole growth carbon source to produce an accumulated quantity of extracellular uniformly labelled $^{13}C$-heteropolysaccharide Poly 54.

EP 0 220 951 A2

-76

# BIOCHEMICALS LABELLED WITH STABLE ISOTOPES

## BACKGROUND OF THE INVENTION

Pharmacological studies with drugs and other compounds labelled with stable isotopes have developed in parallel with the rapid advance in gas chromatography-mass spectrometry analytical techniques, and the studies have been directed mainly to an evaluation of pharmaco-kinetics and drug metabolic pathways.

In the broader scope of biochemistry, stable isotopically labelled compounds have been utilized increasingly in conjuction with both NMR spectroscopy and mass spectroscopy in tracer and structural studies.

One of the most important applications for stable isotopes is the study of metabolic pathways in humans. There is a vast potential for the beneficial application of stable isotopes in the diagnosis of disease and its clinical management.

One of the dominant themes in the development of stable isotope methodology for metabolic studies has been concern for the population of infants, children and pregnant women who are denied the benefit of radioactive isotopic diagnostic procedures because of the associated radiation exposure risk.

A series of $^{13}CO_2$ breath tests have been developed for the early detection of metabolic disease or organ dysfunction such as fat malabsorption or diabetes. This area of medical technology is reviewed by D. M. Glaubitt in Proc. 22nd Int. Conf. Stable Isotopes (Proc. Conf. Oak Brook, 1975)

A major obstacle in the development of stable isotop biomedical applications is the fact that appropriate $^{13}C$-labelled substrates are either not available or are too expensive.

Accordingly, it is an object of this invention to provide a process for efficient production of stable isotopically labelled organic compounds.

It is another object of this invention to provide biochemicals which are labelled with $^{13}C$ isotopic atoms.

It is a further object of this invention to provide uniformly labelled $^{13}C$-glucose.

Other objects and advantages of the present inventioi shall become apparent from the accompanying description and examples.

## DESCRIPTION OF THE INVENTION

One or more objects of the present invention are accomplished by the provision of a process for producing isotope labelled biochemicals which comprises cultivating a methylotrophic microorganism in a nutrient medium containing a a growth carbon source comprising a $^{13}C_1$-compound.

The growth carbon source can comprise a single $^{13}C_1$-compound, or a mixture of $^{13}C_1$-compounds, or a mixture of $^{13}C_1$-compound and unlabelled growth carbon compound, or one or more growth carbon compounds which contain $^{13}C$ and one or more other stable isotope atoms such as $^{18}O$, $^{15}N$ and D. The other stable isotope atoms such as $^{18}O$, $^{15}N$ and D can be contained in assimilable inorganic compounds such as $D_2O^{18}$ and $^{15}ND_3$.

In another embodiment, this invention provides a process for producing isotope labelled biochemicals which comprises aerobically cultivating a Methylophilus viscogenes strain in a minimal salts medium containing a growth carbon source comprising a $^{13}C_1$-compound to produce $^{13}C$-labelled biomass.

In another embodiment, this invention provides a process for producing isotope labelled biochemicals which comprises aerobically cultivating a Methylophilus viscogenes strain in a minimal salts medium containing a growth carbon source comprising a $^{13}C_1$-compound to produce $^{13}C$-labelled

biomass and an accumulated quantity of an extracellular $^{13}C$-labelled biochemical, e.g., a $^{13}C$-labelled polysaccharide.

In another embodiment, this invention provides a process for producing an isotope labelled exopolysaccharide which comprises aerobically cultivating a Methylophilus viscogenes strain in an aqueous minimal salts medium containi $^{13}C$-compound (e.g., $^{13}C$-methanol) as the sole growth carbon source to produce an accumulated quantity of an extracellular uniformly labelled $^{13}C$-heteropolysaccharide.

In another embodiment, this invention provides a process for producing an isotope labelled heteropolysaccharid which comprises aerobically cultivating Methylophilus viscoge strain ATCC 39893 or a variant thereof in a nutrient medium containing a growth carbon source comprising $^{13}C$-methanol to yield $^{13}C$-heteropolysaccharide Poly 54 as an accumulated extracellular product, and optionally recovering the said product from the culture medium.

The production of unlabelled heteropolysaccharide Poly 54 with strain ATCC 39893 is described in copending pate application S.N. 700,564, filed February 11, 1985, incorporat herein by reference.

Illustrative of methylotrophic species suitable for the practice of the present invention are Methylomonas methanica, Methylomonas albus, Methylomonas streptobacterium,

Methylomonas agile, Methylomonas rubrum, Methylomonas rosaceous, Methylobacter chroococcum, Methylobacter bovis, Methylobacter capsulatus, Methylobacter vinelandii, Methylococcus capsulatus, Methylococcus minimus, Methylosinus trichosporium, Methylosinus sporium, Methylobacterium organophilum, Methylocystis parvus OBBP, Methylomonas methylovora, Methylophilus methylotrophus, Methylomonas aminofaciens, Methylomonas methanolica, Pseudomonas extorquens, Protaminobacter ruber, Pseudomonas methylica, Pseudomonas aminovorans, Anthrobacter rufescens, Anthrobacter globiformis, Bacillus cereus, Mycobacterium vaccae, Gliocladium deliquescens, Trichoderma lignorum, Hansenula polymorpha, Candida boidinii, Pichia pinus, Torulopsis pinus, and the like.

Illustrative of a suitable bacterial culture for the invention process embodiments is one having the identifying characteristics of strain ATCC 39893, said culture being capable of aerobic bioconversion of a $C_1$-compound to an extracellular accumulation of heteropolysaccharide.

Subcultures of accession Number ATCC 39893 strain can be obtained upon request from the permanent microorganism collection of the American Type Culture Collection, 12301 Parklawn Drive, Rockville, Maryland 20852. The culture deposit meets the requirements of The Budapest Treaty on patent procedure.

The bacterial strains having the identifying characteristics of strain ATCC 39893 are not members of any of the known methylotrophic species such as *Methylophilus methylotrophus*. For purposes of taxonomic identification, in copending patent application S.N. 700,564, the name *Methylophilus viscogenes* has been assigned to the new facultative methylotroph species which includes bacterial strains having the identifying characteristics of strain ATCC 39893 or variants thereof.

The term "methylotroph" as employed herein refers to microorganism which is able to grow at the expense of reduced carbon compounds having one or more carbon atoms but no carbon-carbon bonds. A "reduced carbon" compound is one havi a lower oxidation state than carbon dioxide.

The term "facultative methylotroph" as employed here refers to a methylotroph which is capable of growth on one or more heterotrophic substrates, e.g., fructose.

The term "$C_1$-compounds" as employed herein refers to organic compounds which do not contain any carbon-carbon bond such as methanol, formaldehyde, formate, formamide, carbon monoxide, dimethyl ether, methylamine, dimethylamine, trimethylamine and trimethylamine N-oxide.

The term "exopolysaccharide" as employed herein refe to a polysaccharide which accumulates as an extracellular metabolite in a fermentation medium, as exemplified by xantha gum and heteropolysaccharide Poly 54.

The term "heteropolysaccharide" as employed herein refers to a polysaccharide which is composed of at least two different kinds of monosaccharidic units, such as glucose and galactose.

The term "isotope labelled biochemical" as employed herein refers to a biochemical which is stable isotope enriched, i.e., the biochemical has a stable isotope atom content greater than the natural stable isotope atom distribution level.

The term "stable isotope" as employed herein refers to non-radioactive isotopes such as D, $^{13}C$, $^{15}N$, $^{18}O$, $^{28}Si$ and $^{34}S$.

The term "ribulose monophosphate pathway"(RMP) as employed herein refers to the biochemical cycle in which three molecules of formaldehyde are condensed to produce either one molecule of pyruvate or one molecule of dihydroxyacetone phosphate.

Biochemical literature relating to elucidation of the ribulose monophosphate pathway and its variations include Biochem. J., 144, 465(1974) by J. Strom et al; Sci., Prog., 62, 167(1975) by C. Anthony; and Biochem. J., 148, 513(1975) by Colby et al.

Strains of *Methylophilus viscogenes* exhibit a novel combination of properties that distinguish them from other methylotrophic bacteria. A *Methylophilus viscogenes* bacterium

such as strain ATCC 39893 is a facultative methylotroph which utilizes the ribulose monophosphate pathway of $C_1$ assimilation, and which typically has a growth rate doubling time of 1-3 hours at 35°-40°C.

Strain ATCC 39893 grows on methanol, fructose and glucose. In addition, it will grow on a wider variety of heterotrophic substrates (e.g., succinate and pyruvate) when an exogenous energy supply in the form of formate or methanol is present. This is a property not previously described for any known microorganism. For purposes of identification, bacteria manifesting this phenomenon are herein termed "latent facultative methylotrophs".

A Methylophilus viscogenes type of bacterial strain can be cultured aerobically in a nutrient medium comprising a carbon source, nitrogen source, and salts.

Suitable nitrogen sources include ammonium sulfate, ammonium chloride, ammonia, diammonium phosphate, ammonium nitrate, sodium nitrate, urea, corn steep liquor, casein, peptone, yeast extract, meat extract, and the like.

Suitable mineral salts include calcium salts, magnesium salts, potassium salts, phosphate salts, iron salts, manganese salts, zinc salts, copper salts, and the like.

Cultivation of a microorganism in a nutrient medium typically is conducted aerobically at a temperature of 25°-50°C and a pH between about 4.0-8.0 by means of shaken or submerged

cultivation. A present invention strain of Methylophilus viscogenes produces and accumulates an exopolysaccharide in a high concentration and with a high rate of carbon source utilization.

Strain ATCC 39893 is capable of producing the exopolysacccharide in an amount up to about 10 grams per liter, based on the culture medium, when methanol is used as the growth carbon source. The yield of exopolysaccharide typically will vary in the range between about 30-60 percent, based on the quantity of methanol utilized.

Stable Isotope-labelled Biochemicals

The cultivation of a methylotrophic microorganism wi[th] a $^{13}C_1$-compound growth carbon source in accordance with the present invention process embodiments yields $^{13}C$-labelled biomass and/or extracellular metabolites such as a $^{13}C$-labelled polysaccharide.

An essential feature of the invention process embodiments is the inclusion of a $^{13}C_1$-compound as a growth carbon source, such as $^{13}C$-carbon monoxide, $^{13}C$-methane, $^{13}C$-methanol, $^{13}C$-formaldehyde, and the like.

If a $^{13}C_1$-compound is the sole growth carbon source, then the resultant biomass and/or extracellular metabolites are uniformly labelled with $^{13}C$ isotope atoms, e.g., uniformly labelled $^{13}C$-heteropolysaccharide Poly 54.

In addition to $^{13}C$ isotope labelling, other stable isotope atoms can be incorporated in the isotope labelled biomass and extracellular metabolites. The incorporation of two or more stable isotope labels in the bioconversion produc[t] is accomplished by the utilization of a $^{13}C_1$-compound growth carbon source which contains one or more additional stable isotopes such as $^{18}O$, $^{15}N$ and deuterium. Illustrative of such growth carbon sources are $CD_3OD$, $^{13}CH_3{}^{15}NH_2$, $^{13}CH_3{}^{18}OH$, $^{13}C^{18}O$, and the like. Optionally, a mixture of $^{13}C_1$-compound growth carbon source and other labelled compounds can be employed such as a mixtur[e] of $^{13}CO$, $^{15}NH_3$ and $^{18}O_2$.

In addition to extracellular isotope labelled metabolites such as $^{13}C$-polysaccharides, the invention process embodiments produce isotope labelled biomass components such as nucleotides, proteins, lipids, lipoproteins, saccharides, aminoacids, and the like.

After the completion of a present invention bacterial cultivation process, the whole cells can be removed from the fermentation broth by conventional means such as centrifugation or filtration. An extracellular polysaccharide is recovered from the supernatant by any convenient procedure such as freeze-drying, or precipitation with a water-soluble organic solvent, e.g., acetone, methanol, ethanol, and the like. Precipitation of the $^{13}C$-polysaccharide can also be effected by treatment of the solution with a calcium salt.

The crude $^{13}C$-polysaccharide can be redissolved in water, and the aqueous solution then subjected to dialysis and lyophilization to provide a purified product.

$^{13}C$-labelled biomass components can be recovered from the whole cells by the conventional procedures which are utilized to separate and recover nucleotides, proteins, and the like.

An isotope labelled metabolite can be produced and then chemically converted into other derivatives. Thus, in another embodiment this invention provides a process for producing isotope labelled saccharides which comprises

cultivating a methylotrophic microorganism in a nutrient mediu containing a growth carbon source comprising a $^{13}C_1$-compound to form an accumulated quantity of an extracellular $^{13}C$-polysaccharide, and reacting the $^{13}C$-polysaccharide in an aqueous medium under hydrolytic pH conditions to produce a reaction product comprising $^{13}C$-saccharides.

When the $^{13}C_1$-compound is the sole source of growth carbon in the above process embodiment, then the produc is a mixture of uniformly labelled $^{13}C$-saccharides. If the $^{13}C$-heteropolysaccharide is uniformly labelled $^{13}C$-heteropolysaccharide Poly 54, then the main constituent of the $^{13}C$-saccharide product mixture is uniformly labelled $^{13}C$-glucose.

The monosaccharide unit composition of uniformly labelled $^{13}C$-heteropolysaccharide Poly 54 is as follows:

| Constituent Sugars(mole %) | |
|---|---|
| $^{13}C$-glucose | 50 |
| $^{13}C$-galactose | 30 |
| $^{13}C$-mannose | 10 |
| $^{13}C$-glucuronic acid | 10 |

Isotope labelled Poly 54 contains no pyruvate or protein, and it contains about one acetyl group per four monosaccharide units.

Typical hydrolysis conditions in the process embodiment are exemplified by a reaction medium pH of about 1-3, a reaction temperature of about 80°C, and a reaction period of about 2-8 hours. The individual $^{13}C$-monosaccharides can be recovered by liquid chromatographic separation.

Additional process embodiments are contemplated in the practice of the present invention.

In another embodiment, this invention provides a bioconversion process which comprises cultivating a methylotrophic microorganism (or cell extract thereof) in a nutrient medium containing an assimilable $C_n$-compound and a $^{13}C_1$-compound to produce an accumulated quantity of $^{13}C$-labelled $C_{n+1}$-condensation product, where n is an integer with a value of at least 2.

In another embodiment, this invention provides a bioconversion process which comprises cultivating a methylotrophic microorganism (or cell extract thereof) in a nutrient medium containing glycine and a $^{13}C_1$-compound as a growth carbon source to produce an accumulated quantity of (3-$^{13}C$)serine product. For example:

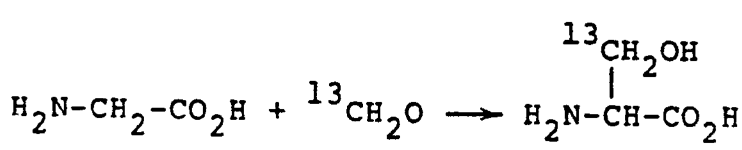

In a further embodiment, this invention provides a bioconversion process which comprises cultivating a methylotrophic microorganism in a nutrient medium containing a carbon isotope-free $^{12}C_1$-compound as a sole growth carbon source to produce $^{12}C$-metabolites which are free of carbon isotope labelling.

Illustrative of a carbon isotope-free growth carbon source is methanol which has been obtained by hydrogenation of carbon isotope-free carbon monoxide ($^{12}CO$). The carbon isotope-free carbon monoxide is produced by high efficiency fractional distillation of normal carbon monoxide which has a 1.1 percent $^{13}C$ isotope content.

When the product is carbon isotope-free $^{12}C$-heteropolysaccharide Poly 54, then by hydrolysis of the Poly 54, a monosaccharide mixture is obtained that has carbon isotope-free $^{12}C$-glucose as the main constituent.

The following examples are further illustrative of th present invention. The components and specific ingredients ar presented as being typical, and various modifications can be derived in view of the foregoing disclosure within the scope o the invention.

For growth of $^{13}C_1$-compound assimilating bacteria a minimal salts medium(MS) (Table I) is employed. The medium is either supplemented with 1 g/L potassium nitrate, giving nitrate mineral salts medium(NMS), or 1 g/L ammonium chloride,

giving ammonium mineral salts medium(AMS). The growth carbon source, e.g., $^{13}C$-methanol, preferably is present at a concentration between 0.2-0.5%(v/v).

For solid media, 17 g/L of Difco bacto-agar is added to the basic mineral salts medium (minus phosphates) prior to sterilization. Sterile phosphate solution is added aseptically to the sterile mineral salts medium on cooling.

Fermentation procedures are conducted in a New Brunswick Microferm (14 liters). A 10 liter working volume is used, and $^{13}C_1$-compound is added aseptically after sterilization of the fermentor. A stirring rate of 200 rpm is routinely used with an air delivery rate of two liters $min.^{-1}$. The fermentor is equipped with pH and dissolved oxygen control.

Hexulose phosphate synthase/hexulose phosphate isomerase are assayed simultaneously, since there is no assay for hexulose 6-phosphate which is the product of hexulose phosphate synthase activity. Hexulose phosphate isomerase converts hexulose 6-phosphate to glucose 6-phosphate which can be estimated using glucose 6-phosphate dehydrogenase and following the concomitant $NADP^+$ reduction at 340 nm. The enzyme assay solution contains (final conc.): phosphate buffer 50 mM, pH 7.2; magnesium chloride 2.5 mM; glucose 6-phosphate dehydrogenase 0.7 units; phosphoglucoisomerase 0.75 units; phosphoribose isomerase 1.75 units; ribose 5-phosphate 5 mM; $NADP^+$ 0.25 mM; and formaldehyde 5 mM.

Any presence of hydroxypyruvate reductase is indicative of the serine pathway of formaldehyde assimilation in the microorganism. This is determined by an enzyme assay solution which contains (final conc.): potassium phosphate, 0.1 M, pH 6.3; lithium hydroxy pyruvate 0.01 M; and NADH 2 mM. NADH disappearance is measured at 340 nm.

The estimation of glycerol and dihydroxyacetone is accomplished with glycerol dehydrogenase/dihydroxyacetone reductase (glycerol:$NAD^+$ 2-oxidoreductase, EC 1.1.1.6). The enzyme is derivated from <u>Enterobacter aerogenes</u>, and is available from Sigma Biochemicals.

For the estimation of glycerol, the reaction mixture (1.0 ml) contains 50 μmole TRIS-HCl buffer (Sigma Biochemicals), pH 9.7; 0.2 μmole $NAD^+$; 1 unit enzyme; and 15 μmole glycerol (or test solution/fermentation broth, 20-50 μl). Assays are initiated by addition of substrate and followed by monitoring increasing absorbance at 340 nm in a Beckman Model 25 UV spectrophotometer.

For the estimation of dihydroxyacetone, the reaction mixture (1.0 ml) contains 50 μmole phosphate buffer, pH 6.0; 0.5 μmole NADH; 1 unit enzyme; 15 μmole dihydroxyacetone (or test solution/fermentation broth, 20-50 μl). Assays are initiated by addition of substrate and followed by monitoring decreasing absorbance at 340 nm.

The isotope labelled exopolysaccharide (e.g., $^{13}C$-heteropolysaccharide Poly 54) is recovered after the completion of a *Methylotrophus viscogenes* strain cultivation run. The broth is removed from the fermentor, and whole cells are removed by centrifugation (13,000 xg for 10 minutes). The exopolysaccharide usually is isolated by isopropanol precipitation from solution.

Analysis of the $^{13}C$-monosaccharide content of an exopolysaccharide is accomplished by a hydrolysis-gas liquid chromatography method. The method involves hydrolyzing the exopolysaccharide in aqueous trifluoroacetic acid solution, then reducing with sodium borohydride and acetylating with acetic anhydride. The polyacetate derivatives of the monosaccharides are then analyzed by gas chromatography in comparison to standards.

General procedures for fractionating microbial biomass into various biochemical entities are described in "Manual Of Methods For General Microbiology", pages 328-364 (American Society For Microbiology, Washington, D.C., 1981).

Recovery of $^{13}C$-labelled nucleotides from whole cells is accomplished by high performance liquid chromatography in accordance with the procedure described in Analytical Biochemistry, 140, 236(1984).

Recovery of $^{13}C$-labelled aminoacids from whole cells is accomplished in accordance with the procedure described in Analytical Chemistry, 52, 617(1980).

NMR spectroscopic analysis of stable isotope labelled compounds is performed in accordance with the procedures described in Science, 205, 160(1979), and "Nuclear Magnetic Resonance in Biochemistry" (Academic Press, London and New York, 1975).

EXAMPLE

This Example illustrates the culturing of a *Methylotrophus viscogenes* strain to form an accumulated quantity of isotope labelled exopolysaccharide metabolite.

A 500 ml inoculum of strain ATCC 39893 is grown employing MS medium, one gram per liter of ammonium chloride, and 0.5% (v/v) $^{13}$C-methanol as a growth carbon source. The flask is incubated at 37°C for two days. A 14 liter New Brunswick Microferm fermentor containing 10 liters of MS medium, one gram per liter of ammonium chloride, and 0.5% (v/v) $^{13}$C-methanol is inoculated with the above culture.

Initial fermentation conditions are as follows: 37°C, agitation 200 rpm, air flow 2 liters/minute, and pH 7.0. The pH is controlled at about 7.0 during the fermentation. $^{13}$C-Methanol is added intermittenly to provide a concentration of 0.5% (v/v) whenever the fermentation medium becomes carbon exhausted. The concentration of $^{13}$C-methanol is determined by gas-liquid chromatography. After 24-36 hours of fermentation, the agitation rate is increased to 400 rpm.

The fermentation is terminated when $^{13}$C-methanol is no longer being utilized by the culture (usually 5-7 days). The highly viscous fermentation broth is then diluted with isopropanol (2:1 alcohol:broth) to facilitate precipitation of the exopolysaccharide. The resulting precipitate is filtered and then washed with 100% isopropanol, and then with 70%

isopropanol. The precipitated exopolysaccharide is then dried at 55°C in a forced air oven. The dried exopolysaccharide subsequently is ground to a powder in a Wiley Mill. The physicochemical properties of the exopolysaccharide product correspond to those of heteropolysaccharide Poly 54 as described in copending patent application S.N. 700,564.

If $^{13}CD_3OD$ is employed as the sole growth carbon source in the presence of 100% $D_2O$, then uniformly labelled D,$^{13}$C-heteropolysaccharide Poly 54 is produced as an exopolysaccharide metabolite.

The whole cell biomass is separated into isotope labelled biochemicals by the use of conventional procedures.

TABLE I

MS MEDIUM

| | |
|---|---|
| $MgSO_4 \cdot 7H_2O$ | 1 g |
| $CaCl_2$ | 0.2 g |
| $Na_2HPO_4$ | 0.33 g |
| $KH_2PO_4$ | 0.26 g |
| FeEDTA | 5.0 mg |
| $Na_2MoO_4 \cdot 2H_2O$ | 2.0 mg |
| $CuCl_2 \cdot 2H_2O$ | 1.0 mg |
| $FeSO_4 \cdot 7H_2O$ | 500 μg |
| $ZnSO_4 \cdot 7H_2O$ | 400 μg |
| $MnCl_2 \cdot 4H_2O$ | 20 μg |
| $H_3BO_4$ | 15 μg |
| $CoCl_2 \cdot 6H_2O$ | 50 μg |
| $NiCl_2 \cdot 6H_2O$ | 10 μg |
| EDTA | 250μ g |
| $H_2O$ | 1 liter pH 6.8 |

WHAT IS CLAIMED IS:

1. A process for producing isotope labelled biochemicals which comprises cultivating a methylotrophic microorganism in a nutrient medium containing a growth carbon source comprising a $^{13}C_1$-compound.

2. A process in accordance with claim 1 wherein the growth carbon source comprises a mixture of $^{13}C$-labelled $C_1$ and unlabelled carbon compounds.

3. A process in accordance with claim 1 wherein the growth carbon source is $^{13}C$-methanol.

4. A process for producing isotope labelled biochemicals which comprises aerobically cultivating a Methylophilus viscogenes strain in a minimal salts medium containing a growth carbon source comprising a $^{13}C_1$-compound to produce $^{13}C$-labelled biomass.

5. A process in accordance with claim 4 wherein the biomass is subjected to separation procedures to yield constituent $^{13}C$-labelled biochemicals as separate products.

6. A process for producing isotope labelled biochemicals which comprises aerobically cultivating a Methylophilus viscogenes strain in a minimal salts medium containing a growth carbon source comprising a $^{13}C_1$-compound to produce $^{13}C$-labelled biomass and an accumulated quantity of an extracellular $^{13}C$-labelled biochemical.

7. A process in accordance with claim 6 wherein the extracellular biochemical is a $^{13}C$-labelled polysaccharide.

8. A process in accordance with claim 6 wherein the Methylophilus viscogenes strain has the identifying characteristics of ATCC 39893 or a variant thereof.

9. A process in accordance with claim 6 wherein the growth carbon source has a content of $^{13}C$ and at least one other stable isotope.

10. A process in accordance with claim 6 wherein the growth carbon source contains $^{18}O$ isotope atoms.

11. A process in accordance with claim 6 wherein the growth carbon source contains $^{15}N$ isotope atoms.

12. A process in accordance with claim 6 wherein the growth carbon source contains deuterium atoms.

13. A process in accordance with claim 6 wherein the growth carbon source is $^{13}C$-methanol.

14. A process in accordance with claim 6 wherein the cultivation medium contains one or more assimilable inorganic compounds which have a content of stable isotope atoms.

15. A process for producing an isotope labelled exopolysaccharide which comprises aerobically cultivating a Methylophilus viscogenes strain in an aqueous minimal salts medium containing $^{13}C_1$-compound as the sole growth carbon source to produce an accumulated quantity of an extracellular uniformly labelled $^{13}C$-heteropolysaccharide.

16. A process in accordance with claim 15 wherein the Methylophilus viscogenes strain is ATCC 39893 or a variant thereof.

17. A process in accordance with claim 15 wherein the sole growth carbon source is $^{13}C$-methanol.

18. A process in accordance with claim 15 wherein the sole growth carbon source is $^{13}CD_3OD$ and the aqueous medium is essentially 100% $D_2O$, and wherein the heteropolysaccharide product is uniformly labelled with $^{13}C$ and D isotope atoms.

19. A process in accordance with claim 15 wherein the heteropolysaccharide product is uniformly labelled $^{13}C$-heteropolysaccharide Poly 54.

20. A process for producing isotope labelled saccharides which comprises cultivating a methylotrophic microorganism in a nutrient medium containing a growth carbon source comprising a $^{13}C_1$-compound to form an accumulated quantity of an extracellular $^{13}C$-polysaccharide, and reacting the $^{13}C$-polysaccharide in an aqueous medium under hydrolytic pH conditions to produce a reaction product comprising $^{13}C$-saccharides.

21. A process in accordance with claim 20 wherein the microorganism is a bacterial strain having the identifying characteristics of Methylophilus viscogenes strain ATCC 39893 or a variant thereof.

22. A process in accordance with claim 20 wherein the $^{13}C_1$-compound is the sole source of growth carbon, and the product is a mixture of uniformly labelled $^{13}C$-saccharides.

23. A process in accordance with claim 22 wherein the main constituent of the saccharide product mixture is uniformly labelled $^{13}C$-glucose.

24. A bioconversion process which comprises cultivating a methylotrophic microorganism in a nutrient medium containing an assimilable $C_n$-compound and a $^{13}C_1$-compound to produce an accumulated quantity of $^{13}C$-labelled $C_{n+1}$-condensation product, where n is an integer with a value of at least 2.

25. A bioconversion process in accordance with claim 24 wherein a cell extract of the methylotrophic microorganism is employed to provide enzymatic activity.

26. A bioconversion process which comprises cultivating a methylotrophic microorganism in a nutrient medium containing glycine and a $^{13}C_1$-compound to produce an accumulated quantity of (3-$^{13}C$)serine product.

27. A bioconversion process in accordance with claim 26 wherein a cell extract of the methylotrophic microorganism is employed to provide enzymatic activity.

28. A bioconversion process which comprises cultivating a methylotrophic microorganism in a nutrient medium containing a carbon isotope-free $^{12}C_1$-compound as a sole growth carbon to produce $^{12}C$-metabolites which are free of carbon isotope labelling.

29. $^{13}C$-heteropolysaccharide Poly 54.

30. D,$^{13}C$-heteropolysaccharide Poly 54.

31. $^{18}O$,$^{13}C$-heteropolysaccharide Poly 54.

32. $^{15}N$,$^{13}C$-heteropolysaccharide Poly 54.

33. Carbon isotope-free $^{12}C$-heteropolysaccharide Poly 54.

34. Carbon isotope-free $^{12}C$-glucose.